# EUROPÄISCHE PATENTSCHRIFT

(11) **EP 2 877 114 B1**
(45) Veröffentlichungstag und Bekanntmachung des Hinweises auf die Patenterteilung: **28.08.2019**
(21) Anmeldenummer: 13734760.5
(22) Anmeldetag: 08.07.2013
(51) Int. Cl.: A61B 90/30, H05B 37/02, G01J 1/32

(54) **VERFAHREN ZUR AUSLEUCHTUNG EINES AUSLEUCHTBEREICHS UND AUSLEUCHTVORRICHTUNG**
METHOD FOR THE ILLUMINATION OF AN ILLUMINATION AREA AND ILLUMINATION DEVICE
PROCÉDÉ POUR L'ÉCLAIRAGE D'UNE ZONE ÉCLAIRÉE ET DISPOSITIF D'ÉCLAIRAGE

(30) Priorität: 25.07.2012 DE 102012014716
(43) Veröffentlichungstag der Anmeldung: 03.06.2015
(73) Patentinhaber: Drägerwerk AG & Co. KGaA, 23558 Lübeck (DE)
(72) Erfinder: SATTLER, Frank, 23560 Lübeck (DE); FORNASIERO, Livio, 23847 Bliestorf (DE); STARK, Hartmut, 23617 Stockelsdorf (DE)
(74) Vertreter: Mildner, Volker
(86) Internationale Anmeldenummer: PCT/EP2013/064357
(87) Internationale Veröffentlichungsnummer: WO 2014/016104

(56) Entgegenhaltungen:
- DE-A1- 4 122 531
- US-A1- 2003 185 009
- US-A1- 2004 129 860
- US-A1- 2005 219 167
- US-A1- 2009 261 759

## Beschreibung

Die vorliegende Erfindung betrifft ein Verfahren zur Ausleuchtung eines Ausleuchtbereichs, eine Ausleuchtvorrichtung für die Ausleuchtung eines Ausleuchtbereichs sowie die Verwendung einer entsprechenden Ausleuchtvorrichtung oder eines entsprechenden Verfahrens.

Verfahren für die Verbesserung von Ausleuchtbereichen und entsprechende Ausleuchtvorrichtungen sind grundsätzlich bekannt. Sie werden z.B. für die Beleuchtung von Operationsfeldern bei chirurgischen Eingriffen eingesetzt. Solche Ausleuchtvorrichtungen sind häufig mit zumindest zwei Lichtmodulen versehen, welche die gewünschte Ausleuchtung in Richtung des Operationsfeldes zur Verfügung stellen. Das Operationsfeld ist somit zumindest abschnittsweise überlappend mit dem Ausleuchtbereich einer solchen Ausleuchtvorrichtung.

Wenn der Nutzer einer Ausleuchtvorrichtung sich innerhalb des Ausleuchtbereichs bewegt, um dort zu arbeiten, wird er durch seine Körperteile, z.B. Kopf, Rücken oder Arm, einen Teil des emittierten Lichts einzelner Lichtmodule abdecken. Diese Abdeckung führt zu einer Verschattung bzw. einer Teilverschattung des Ausleuchtbereichs, sodass die Helligkeit des Ausleuchtbereichs abnimmt. Dies führt wiederum zu schlechteren Sichtverhältnissen, welche die Arbeit dieser Person erschweren. Insbesondere bei Verwendung für Chirurgen und die Ausleuchtung von Operationsfeldern ist dies von großem Nachteil, da eine hohe Eingriffsqualität des chirurgischen Eingriffs mit einer guten Ausleuchtung einhergeht.

Es wurde bereits grundsätzlich vorgeschlagen, über Näherungssensoren mögliche Hindernisse in Richtung des Ausleuchtbereichs festzustellen und entsprechend eine Regelung der Lichtmodule durchzuführen. Nachteil dieser bekannten Technik ist jedoch, dass die Näherungssensoren über keine wesentliche Richtungscharakteristik verfügen und so eine Ortung von zwei oder mehr Hindernissen in der Nähe der Lichtmodule zu Fehlern führen kann. Zum Beispiel zeigen die DE 10 2008 019 191 A1 oder die EP 1 433 998 B1 Möglichkeiten der Ausleuchtung mit einer Schattenkompensation. Dies kann sowohl eine nur geringfügige Verbesserung der Ausleuchtung als auch eine fehlerhafte Ausleuchtung des Ausleuchtbereiches zur Folge haben.

In der US 2004/129860 A1 wird eine Beleuchtungseinheit offenbart, in der die Detektion eines Objektes innerhalb des Strahlengangs der Beleuchtungseinheit durch eine Überwachung des auszuleuchtenden Bereichs mittels einer CCD-Kamera realisiert wird. Als Alternative wird ein Näherungssensor zur Erfassung eines solchen Objektes beschrieben.

In der US 2009/261759 A1 wird eine Bleuchtungseinheit offenbart, die einen auszuleuchtenden Bereich mittels CCD-Kamera filmt, um eventuelle Schatten zu detektieren und mittels Justierung einzelner Lichtmodule diesem Schattenwurf entgegenzuwirken.

In der US 2003/0185009 A1 wird ein Beleuchtungssystem offenbart, in dem mehrere Lichtmodule modulierte Lichtstrahlen senden, um einen Ausleuchtbereich möglichst optimal auszuleuchten. Dem Einfluss von Schatten, die durch störende Objekte im Strahlengang der Lichtstrahlen entstehen können, wird hierbei durch einen spziellen Detektor begegnet. Dieser Detektor sendet Sensorstrahlen derart, dass für den Fall, dass ein Objekt in den Ausleuchtbereich eindringt, die Sensorstrahlen detektiert werden, sodass die Lichtstrahlen der Lichtmodule entsprechend der detektierten Sensorstrahlen moduliert werden können.

Es ist Aufgabe der vorliegenden Erfindung, die voranstehend beschriebenen Nachteile zumindest teilweise zu beheben. Insbesondere ist es Aufgabe der vorliegenden Erfindung, ein Verfahren zur Ausleuchtung eines Ausleuchtbereichs, eine Ausleuchtvorrichtung sowie eine Verwendung einer Ausleuchtvorrichtung oder eines Verfahrens zur Verfügung zu stellen, welche in kostengünstiger und einfacher Weise eine sichere Reduktion der Abschattung des Ausleuchtbereichs durch eine Verbesserung der Ausleuchtung bewirken.

Voranstehende Aufgabe wird gelöst durch ein Verfahren mit den Merkmalen des unabhängigen Anspruchs 1, eine Ausleuchtvorrichtung mit den Merkmalen des unabhängigen Anspruchs 9, sowie eine Verwendung einer Ausleuchtvorrichtung oder eines Verfahrens mit den Merkmalen des unabhängigen Anspruchs 13. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit der erfindungsgemäßen Ausleuchtvorrichtung und der erfindungsgemäßen Verwendung und jeweils umgekehrt, sodass bezüglich der Offenbarung zu den einzelnen Erfindungsaspekten stets wechselseitig Bezug genommen wird bzw. werden kann.

Ein erfindungsgemäßes Verfahren dient der Verbesserung der Ausleuchtung eines Ausleuchtbereichs, insbesondere eines Operationsbereichs, einer Ausleuchtvorrichtung. Eine solche Ausleuchtvorrichtung weist zumindest zwei Lichtmodule auf, die mit Leuchtmitteln für die Emission von Licht ausgestattet sind. Ein erfindungsgemäßes Verfahren weist die folgenden Schritte auf:
- Emission einer für das Lichtmodul charakteristischen Lichtart mit vorgegebener Amplitude von jedem Lichtmodul,
- Detektion der reflektierten Amplituden aller charakteristischen Lichtarten,
- Vergleich der detektierten Amplituden für jedes Lichtmodul,
- Verändern der Lichtintensität, wenigstens eines Lichtmoduls auf Basis des Vergleichs der detektierten Amplituden für jedes Lichtmodul.

Durch ein erfindungsgemäßes Verfahren kann auf separate Näherungssensoren verzichtet werden. So weist jedes Lichtmodul eine eigene charakteristische Lichtquelle bzw. ein eigenes charakteristisches Leuchtmittel auf, welches die charakteristische Lichtart aussendet. Unter einer charakteristischen Lichtart ist dabei eine Art von Licht zu verstehen, welche sich eindeutig einem Lichtmodul zuordnen lässt. Dabei können unterschiedliche charakteristische Parameter des Lichtes zum Einsatz kommen. So ist es z.B. möglich, dass für jedes Lichtmodul als charakteristische Lichtart eine eindeutige und damit charakteristische Wellenlänge des Lichts gewählt wird. Bei Verwendung von Lichtquellen mit spektral gleichartigem Emissionsverhalten ist es ebenso möglich, die einzelne Lichtquelle durch Herausfiltern schmaler Spektralbereiche zu markieren. In der vorliegenenden Erfindung wird die Veränderung der Pulsweitenmodulation zur Charakterisierung der Lichtart mit Bezug auf ein eindeutiges Lichtmodul verwendet.

Selbstverständlich ist es möglich, dass für die Emission der charakteristischen Lichtart ein separater Emitter in Form einer LED, zur Verfügung gestellt wird. Da jedoch das Lichtmodul selbst mit Leuchtmitteln ausgestattet ist, kann das charakteristische Leuchtmittel auch Teil der übrigen Leuchtmittel des Lichtmoduls sein. Es ist auch möglich, dass das gesamte Lichtmodul nur Leuchtmittel aufweist, welche in der Lage sind, die charakteristische Lichtart zu emittieren.

Bei einem erfindungsgemäßen Verfahren wird für die Ausleuchtung eines Ausleuchtbereichs die Ausleuchtvorrichtung mit den zumindest zwei Lichtmodulen eingeschaltet. Dabei wird von den Lichtmodulen Licht emittiert, welches den Ausleuchtbereich ausleuchtet. Gleichzeitig wird von jedem Lichtmodul eine charakteristische Lichtart emittiert, welche von einem Detektor oder mehreren Detektoren hinsichtlich der reflektierten Amplituden wahrgenommen wird. Durch einen Vergleich der detektierten Amplituden kann festgestellt werden, wie groß der Verlust über den Verlauf des Lichtweges vom Lichtmodul zum Detektor ist. Dieser Verlust kann wiederum Rückschlüsse erlauben auf mögliche Objekte im Bereich zwischen dem jeweiligen Lichtmodul und dem jeweiligen Ausleuchtbereich. Durch die charakteristische Lichtart kann über den Detektor festgestellt werden, welche charakteristische Lichtart exakt zuordenbar zu welchem Lichtmodul, möglicherweise mit einer reduzierten reflektierten Amplitude oder einer erhöhten reflektierten Amplitude, gehört. Damit kann nicht nur ein grundsätzlicher Rückschluss auf mögliche Objekte im Bereich zwischen Ausleuchtvorrichtung und Ausleuchtbereich gezogen, sondern vielmehr auch ein Rückschluss auf das betroffene Lichtmodul getroffen werden. Erst dadurch wird es möglich, dass eine exakte Regelung der betroffenen Lichtmodule bzw. des einen betroffenen Lichtmoduls (verschattetes oder abgeschattetes Lichtmodul) erfolgt. Im Gegensatz zu bekannten Ausleuchtvorrichtungen kann somit viel gezielter, genauer und vor allem schneller eine Regelung stattfinden, um die Ausleuchtung des Ausleuchtbereichs möglichst konstant zu halten.

Wird beispielsweise bei einer Ausleuchtvorrichtung, welche mit einem erfindungsgemäßen Verfahren geregelt wird, ein Arm eines Chirurgen in den Bereich zwischen dem Ausleuchtbereich und der Ausleuchtvorrichtung eindringen, so erfolgt eine Teilabschattung des Ausleuchtbereichs. Charakteristisches Licht, welches von den jeweiligen Lichtmodulen emittiert wird, trifft nun zumindest von einigen Lichtmodulen auf diesen Arm des Chirurgen auf. Von dem Arm wird es reflektiert und legt damit einen deutlich kürzeren Weg zurück zum Detektor des einzelnen Lichtmoduls. Wird ein einzelnes Lichtmodul bzw. jedes Lichtmodul mit einem eigenen Detektor ausgestattet, so wird nun das Lichtmodul, welches durch den Arm des Chirurgen verschattet wird, eine vergrößerte Amplitude wahrnehmen.

Durch die Abdeckung mit dem Arm werden andere Detektoren, insbesondere Detektoren anderer Lichtmodule, eine geringere Amplitude dieses charakteristischen Lichtes wahrnehmen. Bei der Auswertung durch den Vergleich der detektierten Amplituden wird nun festgestellt, welches Lichtmodul durch den Arm des Chirurgen abgeschattet ist. Anschließend erfolgt in erfindungsgemäßer Weise eine Veränderung der Lichtintensität des abgeschatteten Lichtmoduls oder der anderen Lichtmodule.

Bei der Auswertung werden vorzugsweise orthogonal normierte Funktionen verwendet. Insbesondere wird die Detektion der charakteristischen Lichtarten von allen charakteristischen Lichtarten in jedem Detektor durchgeführt. Somit wird jeder Detektor für die Anzahl der Lichtmodule einen Vektor als Ergebnis aufweisen, welcher die Ergebnisse der Amplituden aller charakteristischen Lichtarten und dementsprechend aller Lichtmodule aufweist. Werden die Ergebnisse (Vektorergebnisse) aller Detektoren zusammengeführt, so ergeben sie gemeinsam eine Matrix, die eine Dimension nach Anzahl der Lichtmodule aufweist. Durch Veränderungen bzw. Verschiebungen der Amplituden innerhalb dieser Matrix kann nun ein Rückschluss erfolgen auf die Abschattungsgröße bzw. eine Korrelation zwischen dem abschattenden Objekt und dem zugehörigen Lichtmodul. Im Ergebnis erfolgt in erfindungsgemäßer Weise eine Veränderung der Lichtintensität wenigstens eines Lichtmoduls.

Mit Bezug auf den Vergleich der detektierten Amplituden kann sowohl ein Vergleich mit den emittierten Amplituden aller charakteristischen Lichtarten für jedes Lichtmodul, also auch ein Vergleich mit bereits zeitlich voranstehenden detektierten Amplituden für jedes Lichtmodul erfolgen. So kann zum einen ein Soll/Ist-Vergleich mit den emittierten Amplituden, als auch ein Soll/Soll-Vergleich, also ein temporärer Verlauf der Veränderung der detektierten Amplituden für jedes Lichtmodul im Rahmen der vorliegenden Erfindung erfolgen.

Es kann von Vorteil sein, wenn bei einem erfindungsgemäßen Verfahren die Detektion der reflektierten Amplituden aller charakteristischen Lichtarten an wenigstens zwei verschiedenen Positionen durchgeführt wird. Die Anordnung von entsprechenden Detektoren führt dazu, dass eine verbesserte örtliche Auflösung hinsichtlich der Detektion des Objekts und der Zuordnung zu einem abgeschatteten Lichtmodul durchgeführt werden kann. Dabei kann die Detektion sowohl innerhalb, als auch außerhalb der Lichtmodule stattfinden. Grundsätzlich ist es auch möglich, dass Detektoren im Bereich innerhalb oder um den Ausleuchtbereich herum und damit separat von dem Lichtmodul angeordnet sind.

Erfindungsgemäß ist es ebenfalls möglich, dass bei dem Verfahren die Detektion der reflektierten Amplituden aller charakteristischen Lichtarten in wenigstens zwei Lichtmodulen, insbesondere in allen Lichtmodulen, durchgeführt wird. Mit anderen Worten sind entsprechende Detektoren in einem oder in allen Lichtmodulen vorgesehen. Damit werden die Position der Detektoren und damit auch die Position der Detektion, also des abschattenden Objekts, noch genauer definiert beziehungsweise mathematisch einfacher möglich. Hinsichtlich einer Anordnung von Detektoren in allen Lichtmodulen wird dementsprechend eine Messung möglich, die als Ergebnis für jeden Detektor einen N-dimensionalen Vektor ergibt. Unter N-dimensional ist dabei der Bezug auf eine Anzahl N von Lichtmodulen zu verstehen. Die Vektoren werden in einer Matrix über alle Detektoren zusammengeführt und in bereits beschriebener, erfindungsgemäßer Weise ausgewertet. So kann über einen Vergleich der Veränderung dieser Matrix über die Zeit eine Veränderung der Abschattungssituation ausgewertet werden. Selbstverständlich kann auch ein Vergleich mit Emissionsamplituden und dementsprechend mit einer emittierten Matrix stattfinden, um in erfindungsgemäßer Weise zu einer Verbesserung der Ausleuchtsituation des Ausleuchtbereichs zu kommen.

Ein Vorteil wird ebenfalls dadurch erzielt, dass bei einem erfindungsgemäßen Verfahren durch den Vergleich der detektierten Amplituden für jedes Lichtmodul eine lokale Abschattung des Ausleuchtbereichs einem Lichtmodul zugewiesen wird. Dieses Lichtmodul kann auch als abgeschattetes Lichtmodul bezeichnet werden. Damit kann durch die örtliche Korrelation des Emissionspunktes einer charakteristischen Lichtart sowie des Detektionspunktes der charakteristischen Lichtart ein Rückschluss auf den Ort eines verschattenden Objekts getroffen werden. Der Rückschluss von diesem Ort auf ein entsprechend abgeschattetes Lichtmodul erlaubt es, in äußerst gezielter Weise eine Veränderung der Ausleuchtsituation durchzuführen. Insbesondere kann auf Basis dieser Information einer Zuweisung der lokalen Abschattung auf ein Lichtmodul eine gezielte Anpassung der Lichtintensität wenigstens eines Lichtmoduls stattfinden, wie es nachfolgend noch näher spezifiziert wird.

Vorteilhaft ist es ebenfalls, wenn bei dem erfindungsgemäßen Verfahren die Lichtintensität wenigstens eines Lichtmoduls, welches benachbart zu dem Lichtmodul mit der zugewiesenen Abschattung angeordnet ist, erhöht wird. Zusätzlich oder alternativ ist es möglich, dass die Lichtintensität des Lichtmoduls mit der zugewiesenen Abschattung reduziert wird. Dies sind zwei Möglichkeiten, wie eine Veränderung der Lichtintensität wenigstens eines Lichtmoduls durchgeführt werden kann. Diese beiden Möglichkeiten sind sowohl in Kombination, als auch alternativ zueinander einsetzbar. Wird erkannt, dass ein Lichtmodul durch ein Objekt, z.B. den Arm eines Chirurgen, abgeschattet ist, so wirft dieses Objekt einen Schatten in den Ausleuchtbereich. Um diesen Ausleuchtbereich von diesem Schatten zu befreien, erfolgt z.B. eine Erhöhung der Lichtintensitäten der benachbarten Lichtmodule. Sie übernehmen also die Ausleuchtung des Ausleuchtbereichs in zusätzlicher Weise. Damit wird die Abschattung überlagert bzw. eliminiert, sodass die Ausleuchtintensität im Ausleuchtbereich wieder erhöht wird bzw. im Wesentlichen konstant gehalten wird. Gleichzeitig oder zusätzlich kann die Lichtintensität des abgeschatteten Lichtmoduls reduziert werden. Damit wird einerseits die Leistungsaufnahme reduziert und damit die Effizienz der Ausleuchtvorrichtung verbessert und andererseits eine Erhitzung des Abschattungsgegenstandes (z.B. Kopf) vermieden bzw. deutlich reduziert. Darüber hinaus wird ein mögliches Blenden durch Reflexion vom abschattenden Objekt für den Benutzer vermieden. Durch das Erkennen des abgeschatteten Lichtmoduls wird somit zum einen die Verbesserung der Ausleuchtsituation, zum anderen eine Verbesserung der Blendsituation für den Benutzer eines solchen Verfahrens möglich.

Weiter ist es vorteilhaft, wenn als charakteristische Lichtart für jedes Lichtmodul wenigstens eines der folgenden Unterscheidungsmerkmale eingesetzt wird:
- Wellenlänge des Lichts
- Dimmfrequenz des Lichts
- Pulsweitenmodulation
- Phase

Bei der vorliegenden Erfindung wird als charakteristische Lichtart für jedes Lichtmodul als Unterscheidungsmerkmal die Pulsweitenmodulation eingesetzt. Insbesondere der Einsatz der Pulsweitenmodulation (PWM) ist bei der Verwendung von Leuchtmitteln in Form von LEDs sinnvoll. Dabei wird für die Erzeugung einer bestimmten Lichtintensität die Pulsweite des emittierten Lichtes der LED moduliert. Die Frequenz der Modulation kann dabei als charakteristischer Parameter verwendet werden, um eine eindeutige Zuordnung zu dem jeweiligen Lichtmodul zu erlauben. Der Abstand zwischen den einzelnen Frequenzen der Pulsweitenmodulation als charakteristischer Parameter unterschiedlicher Lichtmodule liegt dabei vorzugsweise im Bereich von mindestens 10 Hz. Damit kann sichergestellt werden, dass von den Detektoren eine eindeutige Separierung der Detektion der einzelnen charakteristischen Lichtarten erfolgen kann. Diese Separierung dient dazu, eine eindeutige Zuordnung der detektierten Amplituden der charakteristischen Lichtart zu dem zugehörigen Lichtmodul zu erlauben. Das charakteristische Licht kann sowohl von einem, als auch von allen Leuchtmitteln des Lichtmoduls ausgesendet werden. Eine besonders einfache Möglichkeit, den Lichtfluss eines einzelnen Leuchtmittels zu erfassen besteht darin, die einzelnen Leuchtmittel nacheinander für definierte Zeiten/Phasen anzuschalten und diese während der Anschaltzeit mit unterschiedlichen Leistungen zu betreiben, die der jeweiligen Helligkeit entsprechen. Es lässt sich dann für jedes Leuchtmittel in der jeweiligen Aktivitätsphase ein Quotient aus Ansteuerleistung und Helligkeit bestimmen, der ein Maß für die Abschattung des Leuchtmittels darstellt. Ebenfalls vorteilhaft ist es, wenn bei einem erfindungsgemäßen Verfahren die charakteristische Lichtart eine Wellenlänge im Bereich zwischen 6 µm und 200 nm aufweist. Insbesondere werden charakteristische Lichtarten im Bereich von Wellenlängen eingesetzt, welche im nicht sichtbaren Bereich für den Menschen liegen. So ist es möglich, dass die charakteristische Lichtart im UV-Bereich bzw. im Infrarotbereich ausgebildet ist. Dies bringt den Vorteil mit sich, dass keine Beeinträchtigung durch die charakteristische Lichtart für die Sichtbarkeitsverhältnisse im Ausleuchtbereich vorliegt.

Ebenfalls vorteilhaft ist es, wenn bei einem erfindungsgemäßen Verfahren die charakteristischen Lichtarten eine sinusförmige Modulation aufweisen, wobei die Phase der sinusförmigen Modulation von Lichtmodul zu Lichtmodul verschoben ist. Damit kann eine sinusförmige Modulation verwendet werden, sodass sich die modulierten Anteile im zeitlichen Mittel aufheben. Dies vereinfacht die Steuerung bzw. Regelung der Modulation und kann vorzugsweise bereits konstruktiv ausgebildet werden, sodass für die Charakterisierung der Lichtart überhaupt nicht mehr gesteuert bzw. geregelt werden muss.

Eine Weiterbildung eines erfindungsgemäßen Verfahrens ist dann vorteilhaft, wenn der Vergleich der detektierten Amplituden für jedes Lichtmodul mehrfach durchgeführt wird und die zeitlichen Ergebnisse ebenfalls miteinander verglichen werden. Dies kann zu einer Altersüberwachung als sekundäre Funktion eines erfindungsgemäßen Verfahrens führen. Selbstverständlich ist es auch möglich, dass die Detektion der Amplituden unabhängig von der Reflexion, als Abzweigung von der jeweils entsprechenden charakteristischen Lichtquelle bzw. dem jeweils charakteristischen Leuchtmittel erfolgt. Diese Abzweigung kann ebenfalls zur Altersüberwachung oder zur Überwachung der emittierten Amplitude eingesetzt werden. Damit kann insbesondere eine Eigendiagnose des Moduls bzw. des Lichtmoduls durchgeführt werden. Auch ist es möglich, eine solche Ausführungsform zur Kalibrierung des Verfahrens und/oder des Lichtmoduls einzusetzen.

Ein weiterer Gegenstand der vorliegenden Erfindung ist eine Ausleuchtvorrichtung für die Ausleuchtung eines Ausleuchtbereichs, aufweisend zumindest zwei Lichtmodule mit jeweils wenigstens einem Leuchtmittel und jeweils zumindest einem charakteristischen Leuchtmittel zur Emission einer charakteristischen Lichtart. Dabei ist zumindest ein Detektor für die Detektion der reflektierten Amplituden aller charakteristischen Lichtarten und zumindest eine Recheneinheit für den Vergleich der detektierten Amplituden vorgesehen. Es findet ein Vergleich der detektierten Amplituden mit den emittierten Amplituden aller charakteristischen Lichtarten statt. Diese Ausleuchtvorrichtung kann einzelne Lichtmodule, z.B. in Form einer Operationsleuchte aufweisen. Die Leuchtmittel sind LEDs. Selbstverständlich ist es auch möglich, dass einzelne Lichtmodule als separate Satelliten vorgesehen sind. Dabei ist es möglich, dass diese Satelliten über eine Regelung hinsichtlich ihrer Ausrichtung veränderbar sind. Diese Ausrichtungsveränderung erfolgt insbesondere in motorischer Weise.

Die Recheneinheit ist ausgebildet für die Veränderung der Lichtintensität wenigstens eines der Lichtmodule auf Basis des Vergleichs der detektierten Amplituden für jedes Lichtmodul. Damit wird eine erfindungsgemäße Ausleuchtvorrichtung vorzugsweise eingesetzt für ein erfindungsgemäßes Verfahren bzw. ist die Recheneinheit ausgebildet für die Durchführung eines erfindungsgemäßen Verfahrens. Damit bringt eine erfindungsgemäße Ausleuchtvorrichtung die gleichen Vorteile mit sich, wie sie ausführlich mit Bezug auf ein erfindungsgemäßes Verfahren erläutert worden sind.

Eine erfindungsgemäße Ausleuchtvorrichtung kann dahingehend weitergebildet sein, dass zumindest zwei Detektoren vorgesehen sind, welche insbesondere jeweils in einem Lichtmodul angeordnet sind. Vorzugsweise sind alle Lichtmodule mit einem eigenen Detektor ausgestattet. Bei einer Anzahl von N Lichtmodulen sind auch N Detektoren vorgesehen. Bei der Detektion ergibt sich also für jeden Detektor ein N-dimensionaler Vektor, wobei beim Zusammenführen aller Vektoren in der Auswertung eine N-dimensionale Matrix die Folge ist. Die Auswertung wurde bereits weiter oben ausführlich erläutert.

Bei einer erfindungsgemäßen Ausleuchtvorrichtung, handelt es sich bei dem zumindest einen charakteristischen Leuchtmittel um eine LED, wobei die charakteristische Lichtart durch Pulsweitenmodulation erzeugt wird. Die unterschiedliche Pulsweitenmodulation ist also das Charakteristikum bzw. der charakteristische Parameter bei der erfindungsgemäßen Ausleuchtvorrichtung. Die Pulsweitenmodulation wird dabei vorzugsweise im Bereich um eine Pulsweitenmodulationsfrequenz von 300 Hertz moduliert. Die Abstände zwischen den einzelnen charakteristischen Pulsweitenmodulationen sind vorzugsweise größer als 10 Hertz.

Ebenfalls Gegenstand der vorliegenden Erfindung ist die Verwendung einer Ausleuchtvorrichtung mit den Merkmalen gemäß der vorliegenden Erfindung oder eines Verfahrens mit den Merkmalen der vorliegenden Erfindung für eine Operationsleuchtvorrichtung. Eine solche Operationsleuchte kann von einem Arzt bzw. einem Chirurgen eingesetzt werden, wobei der Ausleuchtbereich sich im Bereich des Operationsfeldes befindet. Bei diesem Einsatz ist eine erfindungsgemäße Ausleuchtvorrichtung bzw. ein erfindungsgemäßes Verfahren mit besonders großen Vorteilen behaftet.

Die vorliegende Erfindung wird näher erläutert anhand der beigefügten Zeichnungsfiguren. Die dabei verwendeten Begrifflichkeiten "links", "rechts", "oben" und "unten" beziehen sich auf eine Ausrichtung der Zeichnungsfiguren mit normalen lesbaren Bezugszeichen. Es zeigen schematisch:
- Figur 1: eine erste Ausführungsform einer erfindungsgemäßen Ausleuchtvorrichtung,
- Figur 2: die Ausführungsform der Fig. 1 mit eingezeichneten Strahlengängen,
- Figur 3: die Ausführungsform der Fig. 2 mit einem abschattenden Objekt,
- Figur 4: eine weitere Ausführungsform einer erfindungsgemäßen Ausleuchtvorrichtung,
- Figur 5a: eine schematische Darstellung der Auswertung mit einem ersten verschattenden Objekt,
- Figur 5b: eine schematische Darstellung einer Auswertung mit einem weiteren verschattenden Objekt,
- Figur 6a: eine schematische Darstellung der Lichtintensität mehrerer Lichtmodule ohne verschattendes Objekt und
- Figur 6b: die Darstellung der Figur 6a mit verschattendem Objekt.

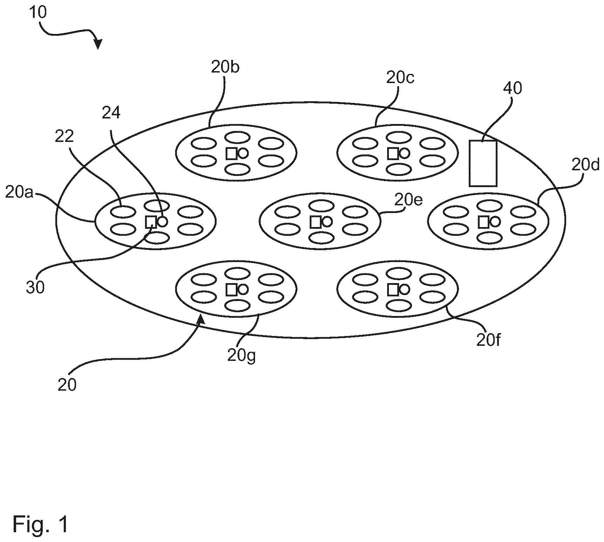

Die Fig. 1 bis 3 zeigen eine erste Ausführungsform einer erfindungsgemäßen Ausleuchtvorrichtung 10. Diese ist mit insgesamt sieben Lichtmodulen 20 versehen, die jeweils mit einzelnen Bezugszeichen 20a, 20b, 20c, 20d, 20e, 20f und 20g bezeichnet sind. Jedes dieser Lichtmodule 20 ist mit einer Vielzahl von Leuchtmitteln 22 versehen, welche insbesondere LEDs sind. Darüber hinaus ist jeweils ein charakteristisches Leuchtmittel 24 für jedes Lichtmodul 20 vorgesehen. Ebenfalls weist jedes Lichtmodul 20 einen Detektor 30 auf. Das charakteristische Leuchtmittel 24 ist eine LED. Darüber hinaus ist eine Recheneinheit 40 vorgesehen, die ein erfindungsgemäßes Verfahren durchführen kann.

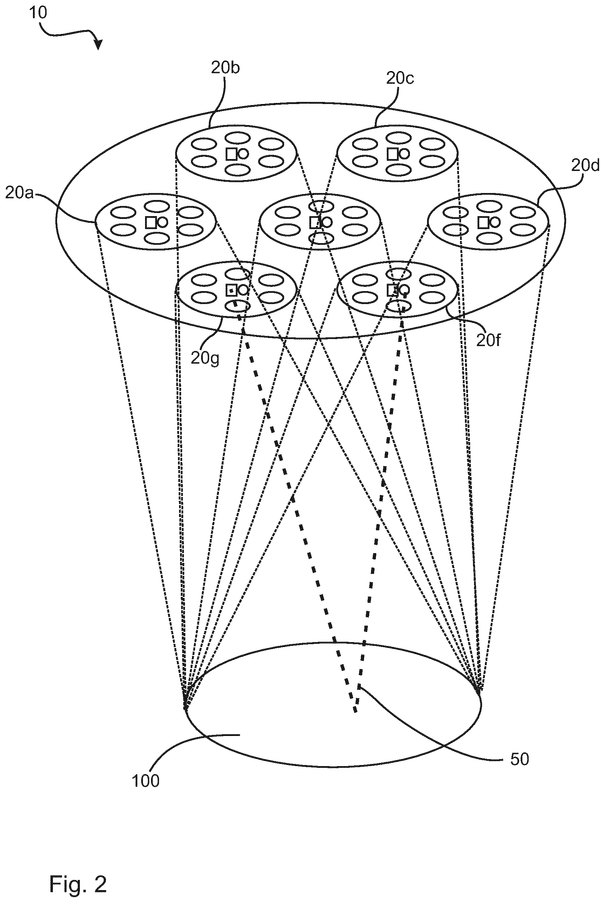

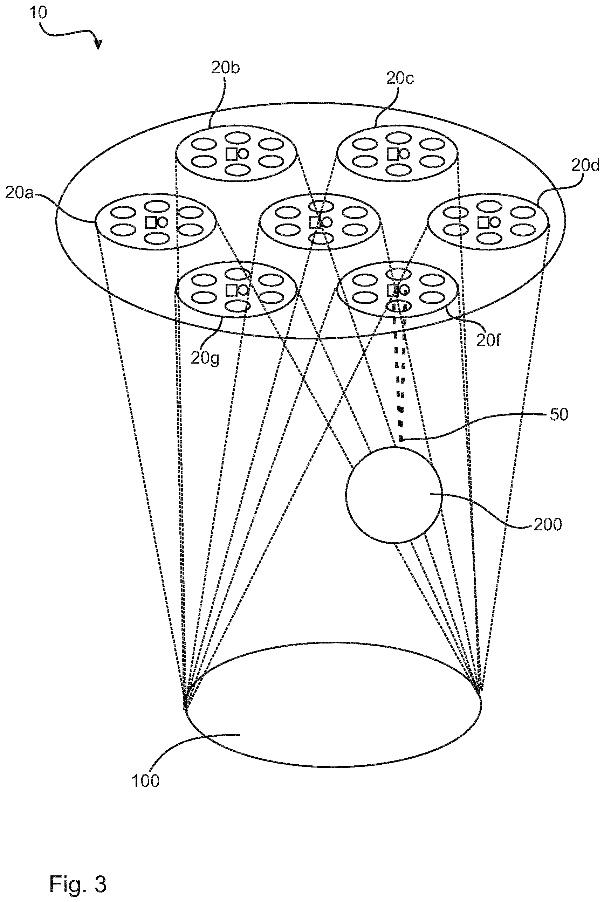

Die Fig. 2 und 3 zeigen grundsätzlich die Funktionsweise eines erfindungsgemäßen Verfahrens mit einer erfindungsgemäßen Ausleuchtvorrichtung 10. So wird ausgehend von jedem Lichtmodul 20 in jedem charakteristischen Leuchtmittel 24 eine charakteristische Lichtart erzeugt. Diese charakteristische Lichtart ist insbesondere hinsichtlich der verwendeten Pulsweitenmodulationen charakteristisch. In Fig. 2 ist eine Situation dargestellt, in welcher alle Lichtmodule 20 ihr Licht auf einen gemeinsamen Ausleuchtbereich 100 werfen. Dieser Ausleuchtbereich 100 ist z.B. das Operationsfeld bei einem Chirurgen. Ein erster Strahlengang ist als Detektionsstrahlengang 50 in Fig. 2 dargestellt, welcher ausgehend vom Lichtmodul 20f bzw. dem dortigen charakteristischen Leuchtmittel 24 auf den Ausleuchtbereich 100 trifft, dort reflektiert wird und von allen Detektoren 30, beispielhaft in Fig. 2, insbesondere von dem Detektor 30 des Lichtmoduls 20g, wieder detektiert wird. Dieser Detektionsstrahlengang 50 ist vielfach zu verstehen und in Fig. 2 nur beispielhaft eingezeichnet. So wird von jedem charakteristischem Leuchtmittel 24 ein Detektionsstrahlengang 50 zu allen anderen Detektoren 30 wie auch zum eigenen Detektor 30 verlaufen. Die Vielzahl der möglichen Strahlengänge erlaubt es nicht, diese einzuzeichnen, da ansonsten die Übersichtlichkeit der Figur verloren gehen würde.

Fig. 3 zeigt die Situation der Fig. 2, nachdem sich ein Objekt 200, z.B. der Arm eines Chirurgen, in den Bereich zwischen dem Ausleuchtbereich 100 und der Ausleuchtvorrichtung 100 bewegt hat. Dieses Objekt 200 verschattet nun einen Teil der Lichtmodule 20 und damit einen Teil des Ausleuchtbereichs 100. Dies ändert den Detektionsstrahlengang 50, wie in Fig. 2 dargestellt. So wird nunmehr der Strahlengang 50 zurück reflektiert auf den eigenen Detektor 30 des Lichtmoduls 20f. Nunmehr fehlt also der Detektionseingang des Detektionsstrahlengangs 50 am Detektor 30 des Lichtmoduls 20g, wie er in Fig. 2 noch vorhanden war. Die entsprechende detektierte Amplitude reduziert sich also am Detektor 30 des Lichtmoduls 20g und erhöht sich am Detektor 30 des Lichtmoduls 20f. Durch die Verschiebung in der entsprechenden Auswertmatrix in der Detektion kann über den Vergleich der einzelnen detektierten Amplituden über die Zeit somit eine Veränderung der Abschattungssituation wahrgenommen werden. Bei der Situation, wie sie Fig. 3 zeigt, ist darüber hinaus eine eindeutige Zuordnung des verschatteten Objekts zum Lichtmodul 20f möglich, da dort eine erhöhte Amplitude und an den anderen Lichtmodulen 20a, 20b, 20c, 20d, 20e und 20g eine reduzierte Amplitude der charakteristischen Lichtart des Lichtmoduls 20f zu erkennen ist.

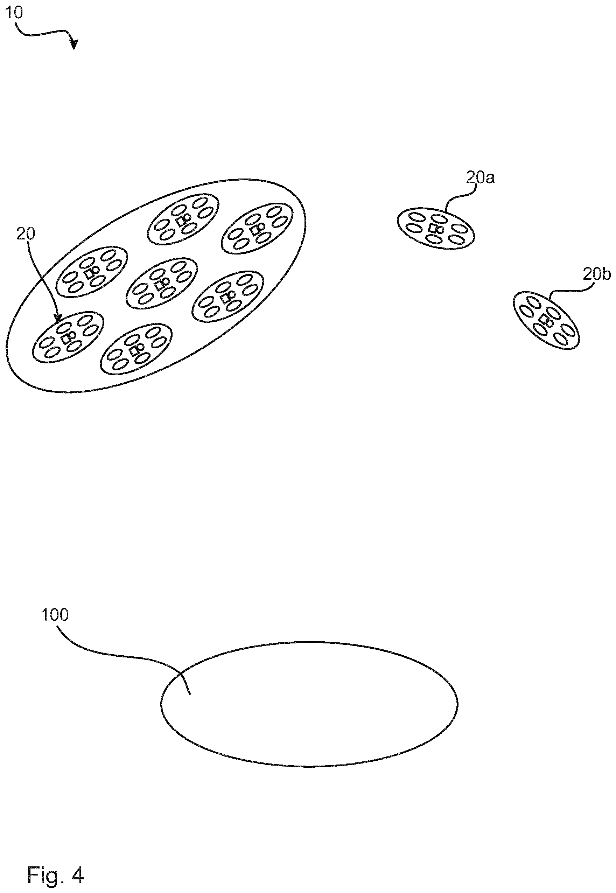

In Fig. 4 ist beispielhaft und schematisch eine weitere erfindungsgemäße Ausleuchtvorrichtung 10 dargestellt. Sie beruht grundsätzlich auf der Ausführungsform, wie sie die Fig. 1 bis 3 zeigen. Jedoch sind zusätzlich zwei weitere Lichtmodule 20a und 20b als Satelliten vorgesehen, die vorzugsweise separat steuerbar sind. Insbesondere sind sie, vorzugsweise motorisch, bewegbar hinsichtlich ihrer Ausleuchtrichtung. Ansonsten funktioniert das Verfahren der Ausleuchtvorrichtung 10 dieser Ausführungsform genau gleich, wie es mit Bezug auf die Fig. 1 bis 3 erläutert worden ist.

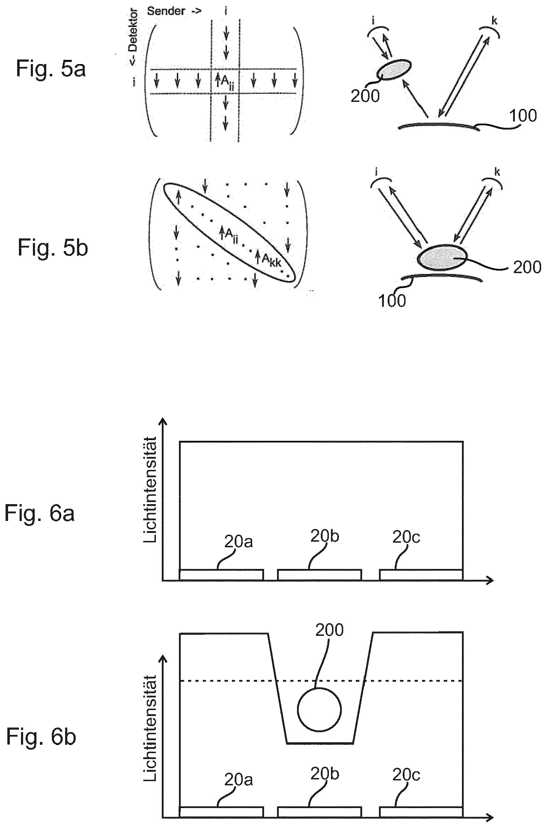

Die Fig. 5a und 5b zeigen schematisch eine Möglichkeit der Auswertung bei einem erfindungsgemäßen Verfahren. In Fig. 5a ist die Situation dargestellt, wie sie grundsätzlich auch die Fig. 3 zeigt. Über einen Ausleuchtbereich 100 erfolgt eine Ausleuchtung von einer Ausleuchtvorrichtung 10 (nicht dargestellt), wobei zwei Detektoren i und k zu erkennen sind. Das Objekt 200 befindet sich im Bereich des Lichtmoduls i, sodass dort eine verstärkte Reflexion stattfindet. Dementsprechend wird in einer Detektionsmatrix, wie sie links in Fig. 5a dargestellt ist, eine charakteristische Amplitudenverteilung für diesen Detektor i hinsichtlich der charakteristischen Lichtart des Lichtmoduls i zu erkennen sein. In Fig. 5b befindet sich das Objekt 200 in anderer Position, sodass sich auch die Reflexionssituation und damit die Detektionssituation verändert. Dies wirkt auf eine Veränderung der Amplituden und damit eine Veränderung der Detektionsmatrix hin, wie sie in Fig. 5b ebenfalls links dargestellt ist. Durch eine entsprechende Auswertung der dargestellten Detektionsmatrizen wird dementsprechend eine besonders eindeutige und genaue Zuordnung des Objekts 200 zu den entsprechenden Lichtmodulen 20 möglich. Insbesondere wird es möglich, unabhängig von der Anzahl der Objekte und von der genauen Position der Objekte, eine besonders vorteilhafte Verbesserung der Ausleuchtung des Ausleuchtbereichs durch entsprechende Regelung der Lichtintensität der einzelnen Lichtmodule 20 durchzuführen.

Die Fig. 6a und 6b zeigen eine grundsätzliche Möglichkeit der Regelung der Lichtintensität. So ist in Fig. 6a die nebeneinander dargestellte Anordnung von drei Lichtmodulen 20a, 20b und 20c dargestellt. Befindet sich kein Objekt 200 in abschattender Position, so kann für alle Lichtmodule 20a, 20b und 20c dieser Ausführungsform die gleiche Lichtintensität verwendet werden. Wird nun ein Objekt 200 in eine abschattende Position bewegt, wie dies Fig. 6b zeigt, so befindet sich dieses in abschattender Position relativ zum mittleren Lichtmodul 20b. Als Strichlinie ist die ursprüngliche Lichtintensität eingezeichnet, wie sie in Fig. 6a dargestellt war. Durch die abschattende Situation des Objekts 200 wird nun das Lichtmodul 20b als abgeschattetes Lichtmodul 20 erkannt. Dementsprechend wird zur Verbesserung der Ausleuchtung die Lichtintensität der benachbarten Lichtmodule 20a und 20c erhöht und gleichzeitig oder alternativ die Lichtintensität des abgeschatteten Lichtmoduls 20b reduziert. Neben einer Reduktion der Blendwahrscheinlichkeit für den Chirurgen wird damit eine Verbesserung der Ausleuchtung erzielt, da sozusagen um das Objekt 200 durch die benachbarten Lichtmodule 20a und 20c herumgestrahlt wird und damit der Schattenwurf vermieden oder verringert wird.

Die voranstehende Erläuterung der Ausführungsformen beschreibt die vorliegende Erfindung nur im Rahmen von Beispielen. Selbstverständlich können einzelne Merkmale der Ausführungsformen, sofern technisch sinnvoll, frei miteinander kombiniert werden.

### BEZUGSZEICHENLISTE

- 10: Ausleuchtvorrichtung
- 20: Lichtmodul
- 20a: Lichtmodul
- 20b: Lichtmodul
- 20c: Lichtmodul
- 20d: Lichtmodul
- 20e: Lichtmodul
- 20f: Lichtmodul
- 20g: Lichtmodul
- 22: Leuchtmittel
- 24: charakteristisches Leuchtmittel
- 30: Detektor
- 40: Recheneinheit
- 50: Detektionsstrahlengang

- 100: Ausleuchtbereich
- 200: Objekt

## Patentansprüche

1. Verfahren zur Ausleuchtung eines Ausleuchtbereichs (100), insbesondere eines Operationsbereichs, einer Ausleuchtvorrichtung (10) mit zumindest zwei Lichtmodulen (20), aufweisend die folgenden Schritte:
- Emission einer für das Lichtmodul (20) charakteristischen Lichtart mit vorgegebener Amplitude von jedem Lichtmodul (20), wobei jedes Lichtmodul (20) eine LED als ein eigenes charakteristisches Leuchtmittel (24) aufweist, welches die charakteristische Lichtart aussendet, wobei die charakteristische Lichtart durch Pulsweitenmodulation erzeugt wird, und wobei unter einer charakteristischen Lichtart eine Art von Licht zu verstehen ist, welche sich eindeutig einem Lichtmodul zuordnen lässt,
- Detektion der Amplituden aller reflektierten, charakteristischen Lichtarten,
- Vergleich der detektierten Amplituden für jedes Lichtmodul (20) mit den emittierten Amplituden für jedes Lichtmodul (20) und/oder mit zeitlich voranstehenden detektierten Amplituden für jedes Lichtmodul (20),
- Verändern der Lichtintensität wenigstens eines Lichtmoduls (20) auf Basis des Vergleichs der detektierten Amplituden für jedes Lichtmodul (20).

2. Verfahren nach Anspruch 1, **dadurch gekennzeichnet, dass** die Detektion der reflektierten Amplituden aller reflektierten, charakteristischen Lichtarten an wenigstens zwei verschiedenen Positionen durchgeführt wird.

3. Verfahren nach einem der vorangegangenen Ansprüche, **dadurch gekennzeichnet, dass** die Detektion der reflektierten Amplituden aller reflektierten, charakteristischen Lichtarten in wenigstens zwei Lichtmodulen (20), insbesondere in allen Lichtmodulen (20), durchgeführt wird.

4. Verfahren nach einem der vorangegangenen Ansprüche, **dadurch gekennzeichnet, dass** durch den Vergleich der detektierten Amplituden für jedes Lichtmodul (20) eine lokale Abschattung des Ausleuchtbereichs (100) einem Lichtmodul (20) zugewiesen wird.

5. Verfahren nach Anspruch 4, **dadurch gekennzeichnet, dass** die Lichtintensität wenigstens eines Lichtmoduls (20), welches benachbart zum dem Lichtmodul (20) mit der zugewiesenen Abschattung angeordnet ist, erhöht wird und/oder die Lichtintensität des Lichtmoduls (20) mit der zugewiesenen Abschattung reduziert wird.

6. Verfahren nach einem der vorangegangenen Ansprüche, **dadurch gekennzeichnet, dass** die charakteristische Lichtart eine Wellenlänge im Bereich zwischen 6 µm und 200 nm aufweist.

7. Verfahren nach einem der vorangegangenen Ansprüche, **dadurch gekennzeichnet, dass** die charakteristischen Lichtarten eine sinusförmige Modulation aufweisen, wobei die Phase der sinusförmigen Modulation von Lichtmodul (20) zu Lichtmodul (20) verschoben ist.

8. Verfahren nach einem der vorangegangenen Ansprüche, **dadurch gekennzeichnet, dass** der Vergleich der detektierten Amplituden für jedes Lichtmodul (20) mehrfach durchgeführt und die zeitlichen Ergebnisse ebenfalls miteinander verglichen werden.

9. Ausleuchtvorrichtung (10) für die Ausleuchtung eines Ausleuchtbereichs (100), aufweisend zumindest zwei Lichtmodule (20) mit jeweils wenigstens einem Leuchtmittel (22) und jeweils zumindest einem charakteristischen Leuchtmittel (24) zur Emission einer charakteristischen Lichtart, wobei unter einer charakteristischen Lichtart eine Art von Licht zu verstehen ist, welche sich eindeutig einem Lichtmodul zuordnen lässt, wobei zumindest ein Detektor (30) für die Detektion der Amplituden aller reflektierten, charakteristischen Lichtarten und zumindest eine Recheneinheit (40) für den Vergleich der detektierten Amplituden mit den emittierten Amplituden für jedes Lichtmodul (20) und/ oder mit zeitlich voranstehenden detektierten Amplituden für jedes Lichtmodul (20) vorgesehen ist,
wobei es sich bei dem zumindest einen charakteristischen Leuchtmittel (24) um eine LED handelt, und wobei die charakteristische Lichtart durch Pulsweitenmodulation erzeugt ist.

10. Ausleuchtvorrichtung (10) nach Anspruch 9, **dadurch gekennzeichnet, dass** die Recheneinheit (40) weiter ausgebildet ist für die Veränderung der Lichtintensität wenigstens eines der Lichtmodule (20) auf Basis des Vergleichs der detektierten Amplituden für jedes Lichtmodul (20).

11. Ausleuchtvorrichtung (10) nach einem der Ansprüche 9 oder 10, **dadurch gekennzeichnet, dass** die Recheneinheit (40) ausgebildet ist für die Durchführung eines Verfahrens mit den Merkmalen eines der Ansprüche 1 bis 8.

12. Ausleuchtvorrichtung (10) nach einem der Ansprüche 9 bis 11, **dadurch gekennzeichnet, dass** zumindest zwei Detektoren (30) vorgesehen sind, welche insbesondere jeweils in einem Lichtmodul (20) angeordnet sind.

13. Verwendung einer Ausleuchtvorrichtung (10) mit den Merkmalen eines der Ansprüche 9 bis 12 oder eines Verfahrens mit den Merkmalen eines der Ansprüche 1 bis 8 für eine Operationsleuchtvorrichtung.

## Claims

1. Method for illuminating an illumination area (100), in particular an operation area, of an illumination device (10) having at least two light modules (20), comprising the following steps:
- emitting a light type that is characteristic of the light module (20) and has a predefined amplitude by each light module (20), wherein each light module (20) comprises an LED as a dedicated characteristic illuminant (24), which emits the characteristic light type, wherein the characteristic light type is generated by pulse width modulation, and wherein a characteristic light type should be understood to mean a type of light which can be assigned unambiguously to a light module,
- detecting the amplitudes of all reflected characteristic light types,
- comparing the detected amplitudes for each light module (20) with the emitted amplitudes for each light module (20) and/or with temporally preceding detected amplitudes for each light module (20),
- altering the light intensity of at least one light module (20) on the basis of comparing the detected amplitudes for each light module (20).

2. Method according to Claim 1, **characterized in that** detecting the reflected amplitudes of all reflected characteristic light types is carried out at at least two different positions.

3. Method according to either of the preceding claims, **characterized in that** detecting the reflected amplitudes of all reflected characteristic light types is carried out in at least two light modules (20), in particular in all the light modules (20).

4. Method according to any of the preceding claims, **characterized in that** as a result of comparing the detected amplitudes for each light module (20), a local shading of the illumination area (100) is allocated to a light module (20).

5. Method according to Claim 4, **characterized in that** the light intensity of at least one light module (20) which is arranged adjacent to the light module (20) with the allocated shading is increased and/or the light intensity of the light module (20) with the allocated shading is reduced.

6. Method according to any of the preceding claims, **characterized in that** the characteristic light type has a wavelength in the range of between 6 µm and 200 nm.

7. Method according to any of the preceding claims, **characterized in that** the characteristic light types have a sinusoidal modulation, wherein the phase of the sinusoidal modulation is shifted from light module (20) to light module (20).

8. Method according to any of the preceding claims, **characterized in that** comparing the detected amplitudes for each light module (20) is carried out repeatedly and the temporal results are likewise compared with one another.

9. Illumination device (10) for illuminating an illumination area (100), comprising at least two light modules (20) each having at least one illuminant (22) and each having at least one characteristic illuminant (24) for emitting a characteristic light type, wherein a characteristic light type should be understood to mean a type of light which can be assigned unambiguously to a light module, wherein at least one detector (30) for detecting the amplitudes of all reflected characteristic light types and at least one computing unit (40) for comparing the detected amplitudes with the emitted amplitudes for each light module (20) and/or with temporally preceding detected amplitudes for each light module (20) are provided,
wherein the at least one characteristic illuminant (24) is an LED, and wherein the characteristic light type is generated by pulse width modulation.

10. Illumination device (10) according to Claim 9, **characterized in that** the computing unit (40) is further configured for altering the light intensity of at least one of the light modules (20) on the basis of comparing the detected amplitudes for each (20).

11. Illumination device (10) according to either of Claims 9 and 10, **characterized in that** the computing unit (40) is configured for carrying out a method having the features of any of Claims 1 to 8.

12. Illumination device (10) according to any of Claims 9 to 11, **characterized in that** at least two detectors (30) are provided, which are arranged in particular respectively in a light module (20).

13. Use of an illumination device (10) having the features of any of Claims 9 to 12 or of a method having the features of any of Claims 1 to 8 for a surgical lighting device.

## Revendications

1. Procédé pour l'éclairage d'une zone éclairée (100), en particulier d'une zone d'intervention chirurgicale, par un dispositif d'éclairage (10) pourvu d'au moins deux modules lumineux (20), présentant les étapes suivantes:
- émission par chaque module lumineux (20) d'un type de lumière caractéristique du module lumineux (20) et d'amplitude prédéfinie, sachant que chaque module lumineux (20) présente une DEL comme propre source lumineuse caractéristique (24) qui émet le type de lumière caractéristique, sachant que le type de lumière caractéristique est produit par modulation de largeur d'impulsions, et sachant qu'on entend par type de lumière caractéristique un type de lumière qui peut être associé de manière univoque à un module lumineux,
- détection des amplitudes de tous les types de lumière caractéristiques réfléchis,
- comparaison des amplitudes détectées pour chaque module lumineux (20) avec les amplitudes émises pour chaque module lumineux (20) et/ou avec des amplitudes détectées antérieures pour chaque module lumineux (20),
- modification de l'intensité lumineuse d'au moins un module lumineux (20) sur la base de la comparaison des amplitudes détectées pour chaque module lumineux (20).

2. Procédé selon la revendication 1, **caractérisé en ce que** la détection des amplitudes réfléchies de tous les types de lumière caractéristiques réfléchis est effectuée en au moins deux positions différentes.

3. Procédé selon l'une des revendications précédentes, **caractérisé en ce que** la détection des amplitudes réfléchies de tous les types de lumière caractéristiques réfléchis est effectuée dans au moins deux modules lumineux (20), en particulier dans tous les modules lumineux (20).

4. Procédé selon l'une des revendications précédentes, **caractérisé en ce que**, par la comparaison des amplitudes détectées pour chaque module lumineux (20), on assigne à un module lumineux (20) un obscurcissement local de la zone éclairée (100).

5. Procédé selon la revendication 4, **caractérisé en ce que** l'intensité lumineuse d'au moins un module lumineux (20) qui est disposé au voisinage du module lumineux (20) auquel est assigné l'obscurcissement est augmentée, et/ou l'intensité lumineuse du module lumineux (20) auquel est assigné l'obscurcissement est réduite.

6. Procédé selon l'une des revendications précédentes, **caractérisé en ce que** le type de lumière caractéristique présente une longueur d'onde dans la plage comprise entre 6 µm et 200 nm.

7. Procédé selon l'une des revendications précédentes, **caractérisé en ce que** les types de lumière caractéristiques présentent une modulation sinusoïdale, sachant que la phase de la modulation sinusoïdale est décalée d'un module lumineux (20) à l'autre.

8. Procédé selon l'une des revendications précédentes, **caractérisé en ce que** la comparaison des amplitudes détectées pour chaque module lumineux (20) est effectuée plusieurs fois, et les résultats dans le temps sont également comparés entre eux.

9. Dispositif d'éclairage (10) pour l'éclairage d'une zone éclairée (100), présentant au moins deux modules lumineux (20) pourvus chacun d'au moins une source lumineuse (22) et chacun d'au moins une source lumineuse caractéristique (24) pour l'émission d'un type de lumière caractéristique, sachant qu'on entend par type de lumière caractéristique un type de lumière qui peut être associé de manière univoque à un module lumineux, sachant qu'il est prévu au moins un détecteur (30) pour la détection des amplitudes de tous les types de lumière caractéristiques réfléchis et au moins une unité de calcul (40) pour la comparaison des amplitudes détectées avec les amplitudes émises pour chaque module lumineux (20) et/ou avec des amplitudes détectées antérieures pour chaque module lumineux (20),
sachant que la source lumineuse caractéristique au moins unique (24) est une DEL, et sachant que le type de lumière caractéristique est produit par modulation de largeur d'impulsions.

10. Dispositif d'éclairage (10) selon la revendication 9, **caractérisé en ce que** l'unité de calcul (40) est en outre réalisée pour la modification de l'intensité lumineuse d'au moins un des modules lumineux (20) sur la base de la comparaison des amplitudes détectées pour chaque module lumineux (20).

11. Dispositif d'éclairage (10) selon la revendication 9 ou 10, **caractérisé en ce que** l'unité de calcul (40) est réalisée pour la mise en oeuvre d'un procédé ayant les caractéristiques d'une des revendications 1 à 8.

12. Dispositif d'éclairage (10) selon l'une des revendications 9 à 11, **caractérisé en ce qu'**il est prévu au moins deux détecteurs (30), qui sont en particulier disposés chacun dans un module lumineux (20).

13. Utilisation d'un dispositif d'éclairage (10) ayant les caractéristiques d'une des revendications 9 à 12 ou d'un procédé ayant les caractéristiques d'une des revendications 1 à 8 pour un dispositif d'éclairage d'intervention chirurgicale.
